# EUROPEAN PATENT APPLICATION

(11) **EP 4 059 444 A2**
(43) Date of publication of application: **21.09.2022**
(21) Application number: 20887745.6
(22) Date of filing: 13.11.2020
(51) Int. Cl.: A61B 10/02, A61B 10/00

(54) **ROLL SHEET-TYPE SAMPLE COLLECTION KIT**

(30) Priority: 15.11.2019 KR 20190146540
(71) Applicant: Lee, Hyungki, Gyeonggi-do 13446 (KR); Kwak, Hwan Jong, Yongin-si, Gyeonggi-do 17103 (KR)
(72) Inventor: Lee, Hyungki, Gyeonggi-do 13446 (KR); Kwak, Hwan Jong, Yongin-si, Gyeonggi-do 17103 (KR)
(74) Representative: Groth & Co. KB
(86) International application number: PCT/KR2020/016039
(87) International publication number: WO 2021/096315

(57) **Abstract**

The present invention relates to a roll sheet type sample collection kit for collecting a sample for a given test, including: a fixing bar provided as a member having a given length; a collection sheet rolledly fixed to one end of the fixing bar to attachedly collect the sample thereonto; a support bar adapted to allow the fixing bar and the collection sheet to protrude from one side thereof and to be grasped while the sample is being collected; and a cap detachably mounted to surround the fixing bar and the collection sheet protruding from the support bar.

According to the present invention, the roll sheet type sample collection kit is capable of making use of a collection sheet as a paper sheet having an excellent absorption force to provide high sample yield, ensuring safe use to minimize a user's psychological reactance, extending a contact area to increase an amount of sample collected and maintain the stable attachment of the sample thereto, thereby performing a sample test in a gentle and accurate manner, and easily removing a portion where the sample is collected therefrom so as to perform the sample test, thereby enhancing quickness and conveniences in the sample test.

## Description

### Technical Field

The present invention relates to a sample collection kit, and more specifically, to a roll sheet type sample collection kit that is capable of providing high sample yield, minimizing a user's psychological reactance owing to safe use thereof, performing a sample test in a gentle and accurate manner, and enhancing quickness and conveniences in the sample test.

### Background Art

Generally, genes used for personal identification are made up of DNA and acquired through profiling, so that they are utilized for various purposes such as personal identity confirmation, paternity confirmation, and the like.

To do such gene profiling, saliva, hair, blood, oral epithelial cells, and the like are collected and used. However, actually, it is hard to collect blood, and after collected, it requires high carefulness when stored. Besides, it is difficult to stably store the collected blood for a long period of time. Also, hair may be contaminated or confused, and further, it is difficult to store the hair for a long period of time. Accordingly, recently, oral epithelial cells have been widely used.

Like this, there is a need to develop a kit capable of easily collecting a sample for gene profiling and stably storing the sample.

To satisfy such a need, a conventional kit for collecting oral cells is disclosed in Korean Patent Application Laid-open No.10-2011-0007809 entitled "Individual gene collection and storage kit". The conventional kit includes a genetic card having a base with a composition for preventing genome deterioration to store genes therein, a sterilized cotton swab for collecting oral epithelial cells, and an envelope made of a UV protection film to store the genes therein.

Because the conventional kit makes use of the cotton swab for collecting oral epithelial cells, however, the collected oral epithelial cells easily escape from the cotton swab, thereby making it hard to gently perform a desired test, having a limitation in increasing sample yield, and causing a user's psychological reactance if the cotton swab is made of a synthetic resin, not a natural material.

Such problems may happen even in collecting samples such as various cells or materials for disease diagnosis as well as in collecting the oral epithelial cells for genetic tests.

### Technical Problem

Accordingly, the present invention has been made in view of the above-mentioned problems occurring in the related art, and it is an object of the present invention to provide a roll sheet type sample collection kit that is capable of making use of a collection sheet as a paper sheet having an excellent absorption force to provide high sample yield, ensuring safe use thereof to minimize a user's psychological reactance, extending a contact area to increase an amount of sample collected and maintain the stable attachment of the sample thereto, thereby performing a sample test in a gentle and accurate manner, and easily removing a portion where the sample is collected therefrom so as to perform the sample test, thereby enhancing quickness and conveniences in the sample test.

### Technical Solution

To accomplish the above-mentioned objects, according to the present invention, there is provided a roll sheet type sample collection kit for collecting a sample for a given test, including: a fixing bar provided as a member having a given length; a collection sheet rolledly fixed to one end of the fixing bar to attachedly collect the sample thereonto; a support bar adapted to allow the fixing bar and the collection sheet to protrude from one side thereof and to be grasped while the sample is being collected; and a cap detachably mounted to surround the fixing bar and the collection sheet protruding from the support bar.

The support bar may have a mounting portion formed at the inside thereof to insertedly mount the fixing bar and a portion of the collection sheet thereonto.

The fixing bar may be a hollow tube.

The fixing bar may have a core inserted into a hollow portion thereof and made of an elastic material.

The collection sheet may have a lower end rolledly attached to the fixing bar, allow a portion excepting the lower end on an exposed end thereof to be unrollably opened, and have a cutoff guide line formed along a lower periphery of the portion exposed from the support bar so as to guide cutoff thereof.

The collection sheet may be rolled to any one of circular, polygonal, and oval sectional shapes on one end of the fixing bar.

### Advantageous Effects

According to the present invention, the roll sheet type sample collection kit can make use of the collection sheet as the paper sheet having an excellent absorption force, thereby providing high sample yield, ensure safe use thereof, thereby minimizing the user's psychological reactance, extend the contact area to increase an amount of sample collected and maintain the stable attachment of the sample thereto, thereby performing the sample test in a gentle and accurate manner, and easily remove the portion where the sample is collected therefrom so as to perform the sample test, thereby enhancing quickness and conveniences in the sample test.

### Brief Description of Drawings

FIG. 1 is an exploded perspective view showing a roll sheet type sample collection kit according to a first embodiment of the present invention.
FIG. 2 is a perspective view showing the roll sheet type sample collection kit according to the first embodiment of the present invention.
FIG. 3 is a perspective view showing a state where a fixing bar and a collection sheet are attached to each other in the roll sheet type sample collection kit according to the first embodiment of the present invention.
FIG. 4 is a perspective view showing a use state of the roll sheet type sample collection kit according to the first embodiment of the present invention.
FIG. 5 is a perspective view showing a use state of a roll sheet type sample collection kit according to a second embodiment of the present invention.
FIG. 6 is a plan view showing various roll shapes of the collection sheet in the roll sheet type sample collection kit according to the present invention.

### Best Mode for Invention

The present invention may be modified in various ways and may have several exemplary embodiments. Specific exemplary embodiments of the present invention are illustrated in the drawings and described in detail in the detailed description. However, this does not limit the invention within specific embodiments and it should be understood that the invention covers all the modifications, equivalents, and replacements within the idea and technical scope of the invention.

Hereinafter, the present invention is disclosed with reference to the attached drawings wherein the corresponding parts in the embodiments of the present invention are indicated by corresponding reference numerals and the repeated explanation on the corresponding parts will be avoided.

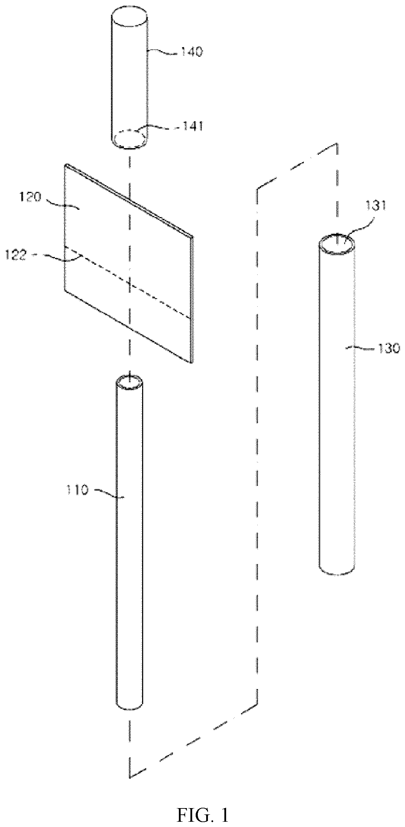

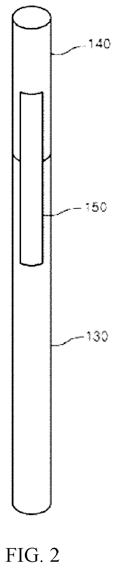

FIG. 1 is an exploded perspective view showing a roll sheet type sample collection kit according to a first embodiment of the present invention, and FIG. 2 is a perspective view showing the roll sheet type sample collection kit according to the first embodiment of the present invention.

Referring to FIGs. 1 and 2, a roll sheet type sample collection kit 100 according to a first embodiment of the present invention includes a fixing bar 110, a collection sheet 120, a support bar 130, and a cap 140 and serves to collect a sample such as oral epithelial cells and various cells or materials needed for genetic tests, disease tests, and other tests through biological and chemical methods.

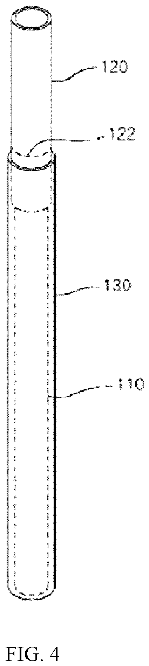

Referring to FIGs. 1, 3 and 4, the fixing bar 110 has a shape of a bar having a given length. For example, the fixing bar 110 is provided to the form of a hollow tube made of a given material, such as a synthetic resin, and the like, thereby achieving lightweight.

The collection sheet 120 is rolled and fixed to one end of the fixing bar 110 and serves to attachedly collect the sample thereto. To do this, the collection sheet 120 may be selected from various sample collection paper sheets including a sample collection paper sheet made by a 3M company and synthetic resin sheets capable of ensuring sample collection.

The collection sheet 120 is provided with an adhesive or a bonding material applied to the lower end thereof so that it can be kept rolled on the fixing bar 110, and accordingly, the collection sheet 120 has an open portion 121 opened to allow a portion excepting the lower end on the exposed end to be unrollable and a cutoff guide line 122 formed along a lower periphery of the portion exposed from the support bar 130 to guide cutoff thereof. In this case, the cutoff guide line 122 may have a cutoff portion continuously formed with a dotted line or has a portion in advance cut off in thickness. Accordingly, the open portion 121 is picked up by tweezers, and next, the collection sheet 120 is cut off along the cutoff guide line 122, thereby enabling a test for the collection sheet 120 to which the sample is attached.

The support bar 130 serves to allow the fixing bar 110 and the collection sheet 120 to protrude from one side thereof and to provide a grasping portion taken by a user while the sample is being collected. The support bar 130 has a mounting portion 131 formed at the inside thereof to be open on one side thereof so that the fixing bar 110 and a portion of the collection sheet 120 can be insertedly mounted on the mounting portion 131. Accordingly, the fixing bar 110 and the collection sheet 120 can be stably fixed to the support bar 130.

Referring to FIGs. 1 and 2, the cap 140 is detachably mounted to surround the fixing bar 110 and the collection sheet 120 protruding from the support bar 130, and according to the present invention, for example, the cap 140 has a sectional area having the same diameter or width as the support bar 130. For example, the cap 140 has an accommodation portion 141 open on one side thereof to open and close the fixing bar 110 and the collection sheet 120 protruding from the support bar 130, and for example, the cap 140 is fitted directly to the collection sheet 120. Otherwise, the cap 140 has one end concavely and convexly coupled or fitted to the support bar 130, while being surroundingly spaced apart from the collection sheet 120.

According to the present invention, a display member 150 is attached to an area on outer surfaces of the support bar 130 and the cap 140, while including a boundary therebetween. Information needed for tests, such as identification codes like barcodes or QR codes, characters, symbols, and the like may be displayed on the display member 150.

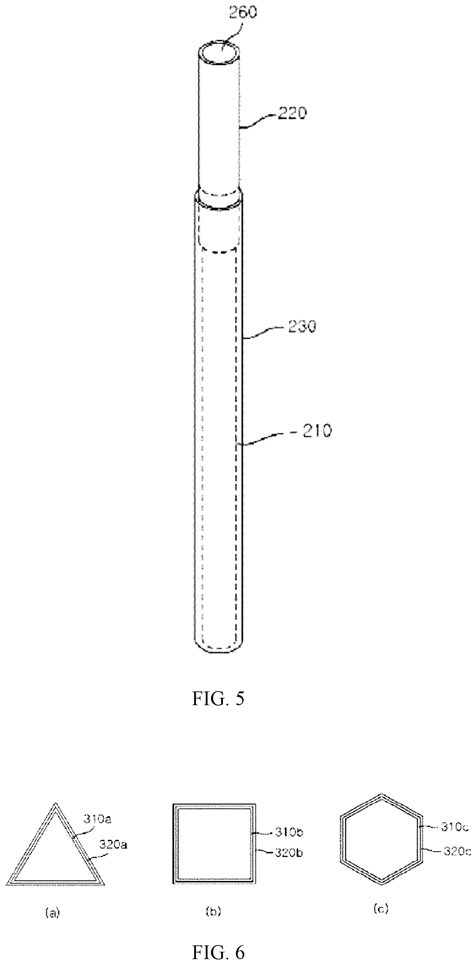

FIG. 5 is a perspective view showing a use state of a roll sheet type sample collection kit according to a second embodiment of the present invention.

Referring to FIG. 5, a roll sheet type sample collection kit according to a second embodiment of the present invention includes a fixing bar 210, a collection sheet 220, a support bar 230, and a cap (not shown), which are the same as the fixing bar 110, the collection sheet 120, the support bar 130, and the cap 140 of the roll sheet type sample collection kit 100 according to the first embodiment of the present invention, and further, the roll sheet type sample collection kit according to the second embodiment of the present invention includes a core 260 disposed inside the fixing bar 210.

In the case where the fixing bar 210 has a shape of a hollow tube, it is convenient to be handled owing to lightweight, but it may be deformed. To solve such a problem, the core 260 is inserted into a hollow portion of the fixing bar 210.

The core 260 is made of an elastic material such as silicone, rubber, flexible synthetic resin, and the like to provide a given restoring force with respect to bending.

FIG. 6 is a plan view showing various roll shapes of the collection sheet in the roll sheet type sample collection kit according to the present invention.

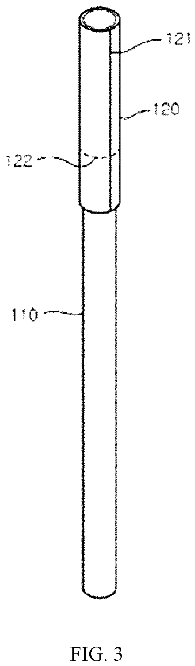

According to the first embodiment of the present invention, the collection sheet 120 is rolled on one end of the fixing bar 110 to the shape of a circle (See FIG. 3), but without being limited thereto, as shown in FIG. 6a, a collection sheet 320a is rolled on one end of a fixing bar 310a to a triangular sectional shape. Further, as shown in FIG. 6b, a collection sheet 320b is rolled on one end of a fixing bar 310b to a square sectional shape, and as shown in FIG. 6c, a collection sheet 320c is rolled on one end of a fixing bar 310c to a hexagonal sectional shape. Otherwise, it may be rolled thereon to an oval sectional shape. Accordingly, the collection sheet may be rolled on the fixing bar to various sectional shapes corresponding to sample collection portions or sample kinds. In this case, the fixing bars 110, 310a, 310b, and 310c have the sectional shapes corresponding to the sectional shapes of the collection sheets 120, 320a, 320b, and 320c, but without being limited thereto, they may have sectional shapes for fixing the collection sheets 120, 320a, 320b, and 320c with various sectional shapes.

According to the present invention, the roll sheet type sample collection kit can make use of the collection sheet as the paper sheet having an excellent absorption force to provide high sample yield and ensure safe use thereof to minimize the user's psychological reactance.

According to the present invention, further, the roll sheet type sample collection kit can extend the contact area to increase an amount of sample collected and maintain the stable attachment of the sample thereto, thereby performing the sample test in a gentle and accurate manner.

According to the present invention, moreover, the roll sheet type sample collection kit can easily remove the portion where the sample is collected therefrom so as to perform a given test, thereby enhancing quickness and conveniences in the sample test.

The foregoing description of the embodiments of the invention has been presented for the purpose of illustration; it is not intended to be exhaustive or to limit the invention to the precise forms disclosed. Persons skilled in the relevant art can appreciate that many modifications and variations are possible in light of the above teachings. It is therefore intended that the scope of the invention be limited not by this detailed description, but rather by the claims appended hereto.

### [Explanations of Reference Numerals]

110, 210, 310a, 310b, 310c: Fixing bar 120, 220, 320a, 320b, 320c: Collection sheet
121: Open portion 122: Cutoff guide line
130, 230: Support bar 131: Mounting portion
140: Cap 141: Accommodation portion
150: Display member 260: Core

## Claims

1. A roll sheet type sample collection kit for collecting a sample for a given test, comprising:
a fixing bar provided as a member having a given length;
a collection sheet rolledly fixed to one end of the fixing bar to attachedly collect the sample thereonto;
a support bar adapted to allow the fixing bar and the collection sheet to protrude from one side thereof and to be grasped while the sample is being collected; and
a cap detachably mounted to surround the fixing bar and the collection sheet protruding from the support bar.

2. The roll sheet type sample collection kit according to claim 1, wherein the support bar has a mounting portion formed at the inside thereof to insertedly mount the fixing bar and a portion of the collection sheet thereonto.

3. The roll sheet type sample collection kit according to claim 1, wherein the fixing bar is a hollow tube.

4. The roll sheet type sample collection kit according to claim 3, wherein the fixing bar has a core inserted into a hollow portion thereof and made of an elastic material.

5. The roll sheet type sample collection kit according to any one of claims 1 to 4, wherein the collection sheet has a lower end rolledly attached to the fixing bar, allows a portion excepting the lower end on an exposed end thereof to be unrollably opened, and has a cutoff guide line formed along a lower periphery of the portion exposed from the support bar so as to guide cutoff thereof.

6. The roll sheet type sample collection kit according to any one of claims 1 to 4, wherein the collection sheet is rolled to any one of circular, polygonal, and oval sectional shapes on one end of the fixing bar.
